(19) Europäisches Patentamt
European Patent Office
Office européen des brevets

(11) Veröffentlichungsnummer: **0 011 601**
**B1**

(12) # EUROPÄISCHE PATENTSCHRIFT

(45) Veröffentlichungstag der Patentschrift: **09.02.83**

(21) Anmeldenummer: **79810142.4**

(22) Anmeldetag: **31.10.79**

(51) Int. Cl.³: **C 07 C 49/747**, C 07 C 49/835, C 07 C 68/00, C 07 C 69/157, C 07 C 69/54, C 07 C 69/63, C 07 C 69/653, C 07 C 79/36, C 07 C 125/06, C 07 C 154/00, A 01 N 31/08

(54) **1H-Inden-1-on-Derivate, Verfahren zu ihrer Herstellung sowie ihre Verwendung in mikrobiziden Mitteln und zur Bekämpfung von Mikroorganismen.**

(30) Priorität: **06.11.78 CH 11528/78**

(43) Veröffentlichungstag der Anmeldung: **28.05.80 Patentblatt 80/11**

(45) Bekanntmachung des Hinweises auf die Patenterteilung: **09.02.83 Patentblatt 83/6**

(84) Benannte Vertragsstaaten: **AT BE CH DE FR GB IT NL**

(56) Entgegenhaltungen:
**Patents Abstracts of Japan Band 2, Nr. 52, April 1978, Seite 270 C 78**
**Chemical Abstracts Band 76, Nr. 21, 1972 Columbus, Ohio, USA A. ROEDIG et al. »Rearrangements of vinylogous carboxylic acid chlorides. X. 1,2-Shift in polychlorinated propenone systems« Seite 444, Spalte 1, Abstract Nr. 126341z in Verbindung mit Chemical Substance Index, Band 76, 1972, Seite 3027CS, Spalte 3, Zeilen 72 bis 75**
**Chemical Abstracts Band 90, Nr. 19, 1979 Columbus, Ohio, USA G. ROBERGE et al. » A convenient synthesis of some highly functionalized chromones and indenones« Seite 589, Spalte 1, Abstract Nr. 151930n in Verbindung mit Chemical Substance Index, Band 90, 1979, Seite 2996CS, Spalte 2, Zeilen 16 bis 17**

(73) Patentinhaber: **CIBA-GEIGY AG, Patentabteilung Postfach, CH-4002 Basel (CH)**

(72) Erfinder: **Sturm, Elmar, Dr., Ziegelbündtenweg 20 B, CH-4147 Aesch (CH)**
Erfinder: **Nyfeler, Robert, Dr., Bärenfelserstrasse 8, CH-4057 Basel (CH)**

**EP 0 011 601 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 1H-Inden-1-on-Derivate, Verfahren zu ihrer Herstellung sowie ihre Verwendung in mikrobiziden Mitteln und zur Bekämpfung von Mikroorganismen

Die vorliegende Erfindung betrifft 2,3-Dichlor-7-hydroxy-1H-inden-1-on-Derivate der Formel I

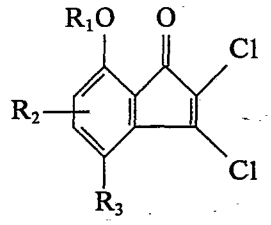

(I)

worin $R_1$ für Wasserstoff oder eine der Gruppen

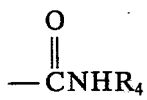 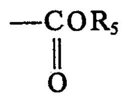 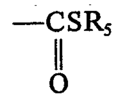 oder 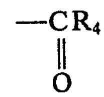

steht und $R_2$ und $R_3$ unabhängig voneinander Wasserstoff, Fluor, Chlor, Brom, $C_1-C_4$-Alkyl, Trifluormethyl oder Nitro, $R_4$ Wasserstoff, unsubstituiertes oder durch Halogen substituiertes $C_1-C_6$-Alkyl, unsubstituiertes oder durch Halogen substituiertes $C_2-C_4$-Alkenyl, unsubstituiertes oder durch $C_1-C_4$-Alkyl, $C_1-C_4$-Alkoxy, Halogen, Trifluormethyl, Cyano oder Nitro substituiertes Phenyl oder aber eine unsubstituierte $C_3-C_6$-Cycloalkylgruppe und $R_5$ $C_1-C_6$-Alkyl, $C_3-C_6$-Cycloalkyl oder $C_2-C_4$-Alkenyl bedeuten.

Als Alkyl oder als Alkylteil eines anderen Substituenten sind je nach Zahl der angegebenen Kohlenstoffatome Methyl, Äthyl, Propyl, Butyl, Pentyl oder Hexyl sowie ihre Isomere wie iso-Propyl, iso-Butyl, sek-Butyl, tert.-Butyl, iso-Pentyl zu verstehen.

Halogen steht für Fluor, Chlor, Brom oder Jod.

Unter Alkenyl sind z. B. Allyl, 2-Butenyl, Methallyl sowie durch Halogen substituierte Gruppen wie Chlorallyl oder Trichlorvinyl zu verstehen. $C_3-C_6$-Cycloalkyl umfaßt Cyclopropyl, Cyclobutyl, Cyclopentyl und Cyclohexyl.

Herstellung und chemische Eigenschaften von 3-Carboxy-2-chlor-7-hydroxy-inden-1-on- und 3-Carboxy-7-hydroxy-indan-1-on-Derivaten werden in Synth. Commun., Band 9, Nr. 2, 1979 (S. 129—139) beschrieben. Eine biologische Aktivität dieser Substanzen wird nicht erwähnt. Ferner werden Indan-2-on-Derivate in der JP-A 53-12421 (Patents Abstracts of Japan, Band 2, Nr. 52, 14. April 1978, S. 270 C 78) als Fungizide offenbart. Diese Substanzen gehören jedoch einer anderen Verbindungsklasse an.

Die Verbindungen der Formel I sind hochwirksame Mikrobizide. Sie können hergestellt werden, indem man

a) eine Verbindung der Formel II

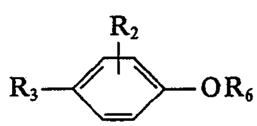

(II)

worin $R_2$ und $R_3$ die für Formel I angegebene Bedeutung haben und $R_6$ Wasserstoff oder bevorzugt Methyl darstellt, mit Trichloracrylsäurechlorid der Formel

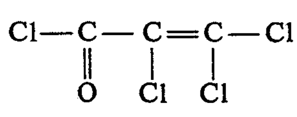

, (III)

in Gegenwart einer Lewis-Säure umsetzt und

b) die so erhaltene Verbindung der Formel IV

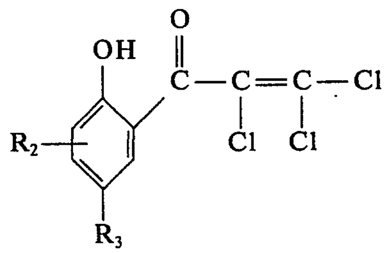

(IV)

ebenfalls in Gegenwart einer Lewis-Säure zu einer Verbindung der Formel V

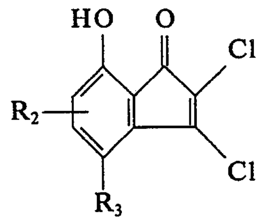

(V)

umsetzt, die einer Verbindung der Formel I entspricht, worin $R_1$ Wasserstoff bedeutet und c) zur Herstellung von Verbindungen der Formel I, worin $R_1$ eine andere Bedeutung als Wasserstoff hat, diese Verbindung der Formel V mit einem, der Bedeutung von $R_1$ entsprechenden Säurehalogenid, Isocyanat, Halogenameisensäureester oder Halogenthioameisensäureester umsetzt.

Die Verfahren werden gegebenenfalls in Gegenwart von gegenüber den Reaktionsteilnehmern inerten Lösungsmitteln durchgeführt wie

- bei Stufe a) z. B.:
  Wasserfreie halogenierte Kohlenwasserstoffe wie Chloroform, Methylenchlorid, Tetrachloräthan, Tetrachlorkohlenstoff oder Schwefelkohlenstoff usw.
- bei Stufe b) z. B.:
  Wasserfreie Schmelze, nicht reaktionsfähige aromatische Kohlenwasserstoffe wie Trichlorbenzol, Nitrobenzol.
- bei Stufe c)
  (i) bei den Säurehalogeniden und Halogensäure(thio)ameisensäureestern aliphatische oder aromatische Kohlenwasserstoffe wie Benzol, Toluol, Xylole, Petroläther; halogenierte Kohlenwasserstoffe wie Chlorbenzol, Methylenchlorid, Äthylenchlorid, Chloroform; Äther und ätherartige Verbindungen wie Dialkyläther, Dioxan, Tetrahydrofuran; Nitrile wie Acetonitril; N,N-dialkylierte Amide wie Dimethylformamid; Dimethylsulfoxid, Ketone wie Methyläthylketon und Gemische solcher Lösungsmittel untereinander. Dabei können säurebindende Mittel wie z. B. die Hydroxide, Carbonate usw. von Alkali- und Erdalkalimetallen oder Stickstoffbasen wie Pyridin und Trialkylamine zugesetzt werden;
  (ii) bei den Isocyanaten wasserfreie Lösungsmittel wie Äther, Tetrahydrofuran, Dioxan, Dimethylformamid und als Katalysator Trialkylamine.

Als Lewis-Säuren kommen z. B. gebräuchliche Vertreter wie Bortrifluorid, Titantetrachlorid, Zinntetrachlorid, EisenIII-chlorid (wasserfrei), Zinkchlorid, vor allem aber Aluminiumtrichlorid in Frage.

Die Reaktionstemperaturen liegen bei Stufe a) zwischen 20 und 120° C, bei Stufe b) zwischen 130 und 180° C und bei Stufe c) zwischen 0 und 180° C. Die Verfahren werden bei Normaldruck durchgeführt.

Durch die Anwendung von zwei Äquivalenten der Lewis-Säure, bezogen auf Säurechlorid und Aromaten, bei Beginn der Reaktion und einer Temperatur von 130—180° C können die zwei Stufen a) und b) zu einem Eintopfverfahren kombiniert werden. Auch hier ist $AlCl_3$ als Lewis-Säure bevorzugt.

In einer Abwandlung des oben beschriebenen Verfahrens läßt sich bei denjenigen Verbindungen der Formel I, worin $R_2$ in der 6-Stellung nicht für Wasserstoff steht, dieser Substituent vorteilhafterweise erst nach Beendigung der Stufe b) in eine Verbindung der Formel Va

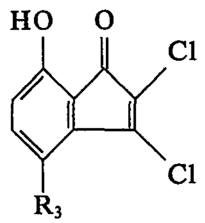

(Va)

nach an sich bekannten Methoden der aromatischen Substitution nachträglich einführen. Dies gilt für Nitro und Halogen.

Bei Verbindungen der Formel II, worin $R_2$ in meta-Stellung steht, verläuft die Friedel-Crafts-Reaktion nicht einheitlich, da sie neben der eigentlichen Verbindung der Formel IV, wenn $R_3 = H$ ist, zwei isomere Verbindungen der Formeln IVa und IVb, und wenn $R_3 \neq H$ eine isomere Verbindung IVa ergibt,

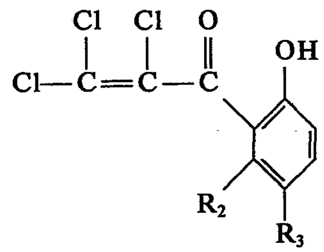

(IVa)

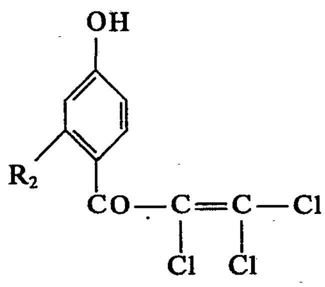

(IVb)

die zu unerwünschten bzw. nicht definierten Endprodukten führen. Ihre Abtrennung aus dem weiteren Reaktionsverlauf erfolgt beispielsweise durch Säulenchromatographie.

Bei Stufe a) des oben beschriebenen Verfahrens können bei Temperaturen unter 100°C bzw. kürzeren Reaktionszeiten (<1 Stunde) definierte Zwischenprodukte der Formel IV isoliert werden.

Erst bei höherer Temperatur, längerer Dauer und Überschuß an Lewis-Säure ($AlCl_3$) entstehen die Verbindungen der Formel V durch innermolekulare Friedel-Crafts-Alkenylierung in dem bereits erwähnten Eintopfverfahren.

Die Verbindungen der Formel IV sind neu, gehören ebenfalls zur Erfindung und weisen eine bemerkenswerte fungizide und bakterizide Wirkung auf. Sie lassen sich ferner zur Herstellung von Polymeren und Copolymeren einsetzen und als Farbstoff-Kupplungskomponenten verwenden.

Herstellung, chemische Eigenschaften und insbesondere intermolekulare Umlagerungserscheinungen strukturähnlicher Phenyltrichlorvinylketone werden in Justus Liebigs Annalen der Chemie, Band 745, 1971, Seiten 35–45, beschrieben. Eine biologische Aktivität der Substanzen wird nicht erwähnt.

Sie können auch durch Reaktion einer Verbindung der Formel VI

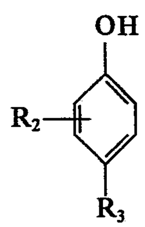

(VI)

in Gegenwart einer Base mit Trichloracrylsäurechlorid erhalten werden.

Dabei entsteht zuerst folgende Verbindung

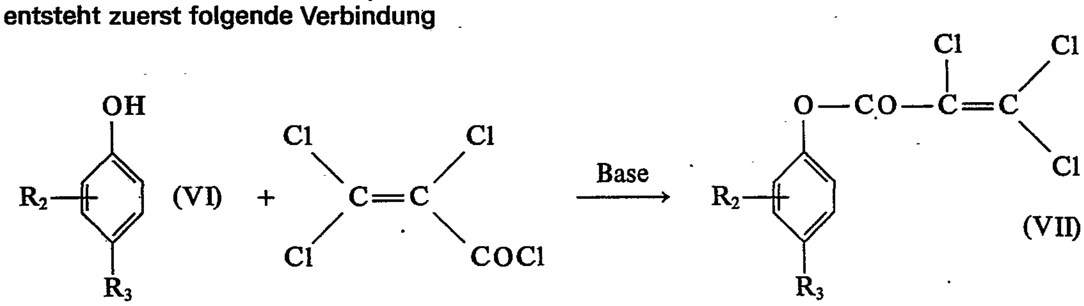


(VII)

Die gewünschte Verbindung IV wird durch eine Fries'sche Verschiebung aus VII in Gegenwart einer Lewis-Säure (bevorzugt Aluminiumtrichlorid) in einem inerten Lösungsmittel erhalten.

Die oben beschriebenen Verfahren bilden gleichfalls einen Teil der Erfindung.

Die substituierten Anisole der Formel II sind handelsübliche Synthetica oder können z. B. aus den entsprechenden Phenolen durch Methylierung mit Dimethylsulfat, Trimethylphosphat o. ä. hergestellt werden.

Die Verbindungen der Formeln I und IV können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen, wie z. B. natürlichen oder regenerierten mineralischen Stoffen, Lösungs-, Dispergier-, Netz-, Haft-, Verdickungs-, Binde- oder Düngemitteln. Die Herstellung solcher Mittel erfolgt in an sich bekannter Weise durch inniges Vermischen und Vermahlen der Bestandteile. Zur Applikation können die Verbindungen der Formeln I und IV in den folgenden Aufarbeitungsformen vorliegen (wobei die Gewichtsprozentangaben vorteilhafte Mengen an Wirkstoff darstellen).

Feste Aufarbeitungsformen:
Stäubemittel, Streumittel, (bis 10%) Granulate, Umhüllungsgranulate, Imprägnierungsgranulate und Homogengranulate; Pellets (Körner) (1 bis 80%)

Flüssige Aufarbeitungsformen:

a) in Wasser dispergierbare
Wirkstoffkonzentrate: Spritzpulver (wettable powders),
Pasten, 25 – 90% in der Handelspackung,
0,01 bis 15% in gebrauchsfertiger Lösung,
Emulsionen; Lösungskonzentrate (10 bis 50%;
0,01 bis 15% in gebrauchsfertiger Lösung)

b) Lösungen: Aerosole

Der Gehalt an Wirkstoff in den oben beschriebenen Mitteln liegt zwischen 0,1 und 95 Gewichtsprozent. Solche Mittel sind gleichfalls Gegenstand dieser Erfindung.

Die Verbindungen der Formeln I und IV können, um sie den gegebenen Umständen anzupassen, selbstverständlich zur Verbreiterung ihres Wirkungsspektrums mit anderen geeigneten Pestiziden, wie z. B. Fungiziden, Bakteriziden, Insektiziden, Akariziden, Herbiziden oder den Pflanzenwuchs beeinflussenden Wirkstoffen zusammen eingesetzt werden. Solche Mittel gehören ebenfalls zur Erfindung.

Die Verbindungen der Formeln I und IV besitzen ein für die praktischen Bedürfnisse sehr günstiges Mikrobizid-Spektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen wie Kartoffeln, Tabak und Tomaten, sowie auch Bananen-, Kakao- und Naturkautschuk-Gewächse.

Mit Verbindungen der Formeln I und IV können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z. B. Venturia, Helminthosporium, Fusarium); Basidiomycetes, wie vor allem Rostpilze (z. B. Puccinia, Tilletia); Fungi imperfecti (z. B. Botrytis, Piricularia, Cercospora) und die der Klasse der Phycomycetes angehörenden Oomycetes wie Plasmopara. Die Wirkstoffe sind auch gegen phytopathogene Bakterien aktiv, wie z. B. Pseudomonas spp., Xanthomonas spp. sowie Erwinia und Corynebacterium. Überdies wirken die Verbindungen zum Teil systemisch. Sie können ferner als Beizmittel zur Behandlung von Saatgut (Früchte, Knollen, Körner) und Pflanzenstecklingen zum Schutz von Infektionen sowie gegen im Erdboden auftretende phytopathogene Mikroben eingesetzt werden. Die vorliegende Erfindung betrifft somit ferner die Verwendung der Verbindungen der Formel I sowie IV zur Bekämpfung von Mikroorganismen.

Folgende Substituententypen sowie Kombinationen dieser untereinander bilden bevorzugte Ausführungen der Erfindung.

$R_1$ = Wasserstoff oder

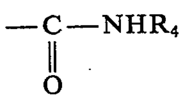

$R_2$ in 6-Stellung Wasserstoff, Nitro oder Halogen,
$R_4$ Methyl oder Äthyl.

Interessant sind auch Verbindungen der Formel I, worin

$R_1$ für Wasserstoff;

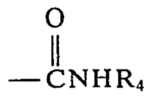 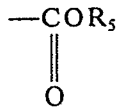 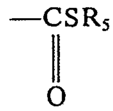 oder 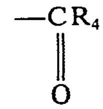

steht,

$R_2$ für Wasserstoff oder (in 6-Stellung) Nitro, Fluor, Chlor oder Brom oder (in 5-Stellung) Fluor, Chlor oder Brom steht,

$R_3$ für Wasserstoff, Fluor, Chlor oder Brom oder $C_1-C_4$-Alkyl steht,

$R_4$ gegebenenfalls halogeniertes $C_1-C_4$-Alkyl oder $C_2-C_3$-Alkenyl bedeutet und

$R_5$ $C_1-C_4$-Alkyl bedeutet.

Die nachfolgenden Beispiele dienen zur näheren Erläuterung der Erfindung, ohne dieselbe einzuschränken. Die Temperaturangaben beziehen sich auf Celsiusgrade, Druckangaben beziehen sich auf Millibar und Teile und Prozentangaben beziehen sich auf Gewicht.

## Herstellungsbeispiele

### Beispiel 1 (Eintopfverfahren)

Herstellung von 2,3-Dichlor-7-hydroxy-4-methyl-1H-inden-1-on der Formel

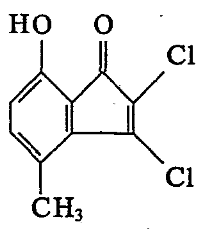

122 g p-Kresol-methyläther und 194 g Trichloracrylsäurechlorid wurden in 500 ml 1,2,4-Trichlorbenzol gelöst. Zu der gerührten Mischung wurden 330 g wasserfreies Aluminiumchlorid in Portionen zugegeben, wobei die Temperatur auf ca. 50° stieg. Danach wurde auf 80° erhitzt, wobei ein gleichmäßiger Chlorwasserstoffstrom entwich. Schließlich wurde die Temperatur auf 120° gesteigert und die Mischung noch 4 Std. gerührt. Nach dem Abkühlen wurde die tiefbraune Reaktionsmischung in Eiswasser eingetragen, wobei sich 140 g gelbbraune Kristalle abschieden. Das Filtrat wurde mit Methylenchlorid extrahiert. Nach Abdampfen des Lösungsmittels und des Trichlorbenzols im Vakuum hinterblieben weitere 60 g Rohprodukt. Die Rohsubstanz wird aus Cyclohexan unter Verwendung von Aktivkohle umkristallisiert. Es wurden 180 g orangerote Kristalle vom Smp. 178—180° erhalten.

### Beispiel 2

Herstellung von 2,3-Dichlor-4-methyl-7-methylcarbamoyloxy-1H-inden-1-on der Formel

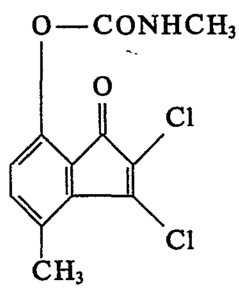

229 g 2,3-Dichlor-7-hydroxy-4-methyl-1H-inden-1-on wurden in einer Mischung von 1 Liter Diäthyläther und 1 Liter Tetrahydrofuran gelöst und 1 ml Triäthylamin zugefügt. Zu dieser Lösung wurden 69 g Methylisocyanat bei 20° zugetropft, wobei die Temperatur auf 28° stieg. Nach ca. 2 Std. setzte Kristallisation ein. Nach 20 Std. wurde der Kristallbrei abgesaugt und mit Diäthyläther gewaschen. Es wurden 220 g orangegelbe Nadeln vom Smp. 189—190° (Zers.) erhalten.

## Beispiel 3

Herstellung von 2,3-Dichlor-7-hydroxy-4-methyl-6-nitro-1H-inden-1-on der Formel

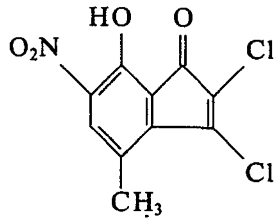

23 g 2,3-Dichlor-7-hydroxy-4-methyl-1H-inden-1-on wurden in 250 ml Eisessig aufgeschlämmt und diese Mischung unter Rühren auf 40° erwärmt. Es wurden dann langsam 10,5 g 65%ige Salpetersäure (d = 1,4) zugetropft, wobei eine tiefgelbe klare Lösung entstand, und das Reaktionsgemisch wurde noch 3 Std. bei 40° weitergerührt. Nach 15stündigem Stehen wurde die Mischung mit 500 ml Wasser verrührt, die gelben Kristalle abgesaugt und noch feucht aus Alkohol umkristallisiert. Es wurden 16 g gelbe Nadeln vom Smp. 168 – 170° erhalten.

## Beispiel 4

a) Herstellung von 5-Brom-2-hydroxyphenyl-trichlorvinylketon (Zwischenprodukt) der Formel

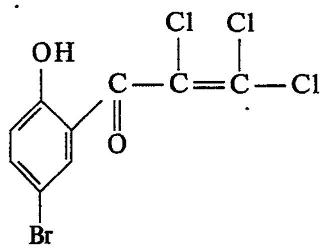

94 g 4-Bromanisol und 110 g Trichloracrylsäurechlorid wurden in 800 ml Dichlormethan gelöst und 99 g wasserfreies Aluminiumchlorid portionenweise zugegeben. Dann wurde die Reaktionsmischung 24 Std. unter Rückfluß erhitzt, wobei Chlorwasserstoff entwich. Nach dem Abkühlen wurde langsam 0,5 N Salzsäure unter stetigem Rühren zugetropft, bis zwei klare Phasen entstanden waren. Die Dichlormethanpase ergab nach dem Eindampfen 130 g eines grünlichen Öls, das im Hochvakuum destilliert wurde. Die Hauptfraktion siedete bei 102 – 105° und 0,02 mbar. Das Destillat erstarrte zu 101 g gelben Kristallen vom Smp. 61 – 64°.

Auf analoge Weise wurden folgende weitere Ausgangsstoffe der Formel IV hergestellt:

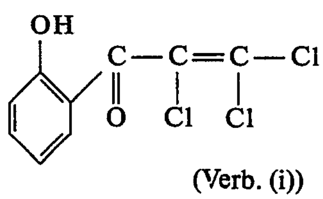

Sdp. 95-100°/0,012 mbar

(Verb. (i))

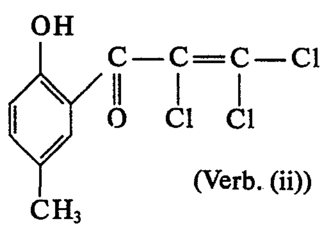

Smp. 55-58°

(Verb. (ii))

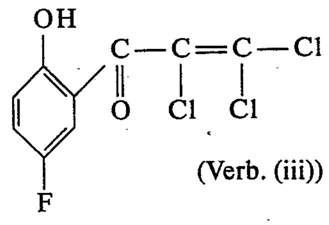

Smp. 59–62°

(Verb. (iii))

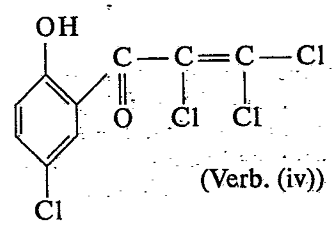

Smp. 43–45°

(Verb. (iv))

b) Herstellung von 4-Brom-2,3-dichlor-7-hydroxy-1H-inden-1-on der Formel

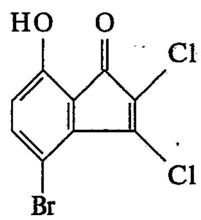

60 g 5-Brom-2-hydroxy-phenyl-trichlorvinyl-keton wurden in 150 ml 1,2,4-Trichlorbenzol gelöst und unter Rühren 33 g Aluminiumchlorid in Portionen zugefügt. Das Reaktionsgemisch wurde zuerst auf 80° und dann allmählich auf 140° erwärmt. Nach 4 Std. wurde die dunkelbraune Masse mit Eiswasser und 0,5 N Salzsäure zersetzt. Nach Extraktion mit Dichlormethan und Abdestillieren des Lösungsmittels im Vakuum wurde eine dunkle halb-kristalline Masse erhalten. Diese Masse wurde mit 250 ml eiskaltem Methanol digeriert, abgesaugt und die braune Kristallmasse aus Cyclohexan/Toluol und Aktivkohle umkristallisiert. Es wurden 20 g hellgelbe Kristalle vom Smp. 170–172° erhalten.

## Beispiel 5

Herstellung von 4-Brom-2,3-dichlor-7-methoxycarbonyloxy-1H-inden-1-on der Formel

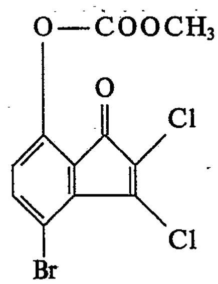

13 g 4-Brom-2,3-dichlor-7-hydroxy-1H-inden-1-on wurden in 200 ml Tetrahydrofuran gelöst, 5 g Triäthylamin zugefügt und zur tiefroten Lösung bei 5–10° 4,5 g Chlorameisensäuremethylester zugetropft. Die Reaktionsmischung wurde noch 3 Std. bei Raumtemperatur gerührt, das Triäthylammoniumchlorid abgesaugt und das Filtrat im Vakuum abgedampft. Die Kristallmasse wurde aus Äthanol umkristallisiert: 12 g gelbe Kristalle vom Smp. 135–137°.

Auf analoge Weise oder nach einer der hierin beschriebenen Methoden können folgende Verbindungen der Formel I hergestellt werden:

Tabelle I

($R_1$ = H)

| Verb. Nr. | $R_2$ | $R_3$ | Physikalische Konstante |
|---|---|---|---|
| 1.1 | H | H | Smp. 147–148° |
| 1.2 | H | $CH_3$ | Smp. 178–180° |
| 1.3 | H | F | Smp. 171–173° |
| 1.4 | H | Cl | Smp. 170–171° |
| 1.5 | H | Br | Smp. 172–173° |
| 1.6 | 6-$NO_2$ | $CH_3$ | Smp. 168–170° |
| 1.7 | 6-Br | $CH_3$ | Smp. 133–135° |
| 1.8 | 6-$NO_2$ | Cl | Smp. 147–149° |
| 1.9 | 6-Cl | Cl | |
| 1.10 | 5-F | H | |
| 1.11 | 5-Cl | H | |

Tabelle II

| Verb. Nr. | $R_2$ | $R_3$ | $R_1$ | Physikalische Konstante |
|---|---|---|---|---|
| 2.1 | H | $CH_3$ | $-CONHCH_3$ | Smp. 190° (Zers.) |
| 2.2 | H | $CH_3$ | —CONH— 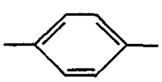 —Cl | Smp. 152-154° |
| 2.3 | H | $CH_3$ | $-CONHC_4H_9$(n) | Smp. 141-143° |
| 2.4 | H | $CH_3$ | $-COOCH_3$ | Smp. 136-138° |
| 2.5 | H | Cl | $-CONHCH_3$ | Smp. 180-182° |
| 2.6 | H | Cl | $-COSC_2H_5$ | Smp. 85-88° |
| 2.7 | H | Cl | $-CO-CH{=}CH_2$ | Smp. 153-155° |
| 2.8 | H | $CH_3$ | $-CO-CCl-CCl_2$ | Smp. 116-118° |
| 2.9 | H | F | $-CONHCH_3$ | Smp. 216-218° |
| 2.10 | H | Br | $-COOCH_3$ | Smp. 135-137° |
| 2.11 | H | Cl | $-COCH_3$ | Smp. 115-117° |
| 2.12 | H | F | $-COCH_2Cl$ | Smp. 143-145° |
| 2.13 | 6-$NO_2$ | $CH_3$ | $-CONH-CH_3$ | Smp. 159-161° |
| 2.14 | H | $CH_3$ | $-CONH-C_2H_5$ | Smp. 186-188° |

## Formulierungsbeispiele

### Stäubemittel

Zur Herstellung eines a) 5%igen und b) 2%igen Stäubemittels werden die folgenden Stoffe verwendet:

| | | |
|---|---|---|
| a) | 5 Teile | Wirkstoff, |
| | 95 Teile | Talkum; |
| b) | 2 Teile | Wirkstoff, |
| | 1 Teil | hochdisperse Kieselsäure, |
| | 97 Teile | Talkum. |

Die Wirkstoffe werden mit den Trägerstoffen vermischt und vermahlen und können in dieser Form zur Anwendung verstäubt werden.

### Granulat

Zur Herstellung eines 5%igen Granulats werden die folgenden Stoffe verwendet:

| | |
|---|---|
| 5 Teile | Wirkstoff, |
| 0,25 Teile | Epichlorhydrin, |
| 0,25 Teile | Cetylpolyglykoläther, |
| 3,50 Teile | Polyäthylenglykol, |
| 91 Teile | Kaolin (Korngröße 0,3 – 0,8 mm). |

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschließend wird das Aceton im Vakuum verdampft. Ein derartiges Mikrogranulat wird vorteilhaft zur Bekämpfung von Bodenpilzen verwendet.

### Spritzpulver

Zur Herstellung eines a) 70%igen, b) 40%igen, c) und d) 25%igen, e) 10%igen Spritzpulvers werden folgende Bestandteile verwendet:

| | | |
|---|---|---|
| a) | 70 Teile | Wirkstoff, |
| | 5 Teile | Natriumdibutylnaphthylsulfonat, |
| | 3 Teile | Naphthalinsulfonsäuren-Phenolsulfonsäuren-Formaldehyd-Kondensat 3 : 2 : 1, |
| | 10 Teile | Kaolin, |
| | 12 Teile | Champagne-Kreide; |
| b) | 40 Teile | Wirkstoff, |
| | 5 Teile | Ligninsulfonsäure-Natriumsalz, |
| | 1 Teil | Dibutylnaphthalinsulfonsäure-Natriumsalz, |
| | 54 Teile | Kieselsäure; |
| c) | 25 Teile | Wirkstoff, |
| | 4,5 Teile | Calcium-Ligninsulfonat, |
| | 1,9 Teile | Champagne-Kreide/Hydroxyäthylcellulose-Gemisch (1 : 1), |
| | 1,5 Teile | Natrium-dibutyl-naphthalinsulfonat, |
| | 19,5 Teile | Kieselsäure, |
| | 19,5 Teile | Champagne-Kreide, |
| | 28,1 Teile | Kaolin; |
| d) | 25 Teile | Wirkstoff, |
| | 2,5 Teile | Isooctylphenoxy-polyoxyäthylen-äthanol, |
| | 1,7 Teile | Champagne-Kreide/Hydroxyäthylcellulose-Gemisch (1 : 1), |
| | 8,3 Teile | Natriumaluminiumsilikat, |
| | 16,5 Teile | Kieselgur, |
| | 46 Teile | Kaolin; |

e) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

## Emulgierbare Konzentrate

Zur Herstellung eines 25%igen emulgierbaren Konzentrats werden folgende Stoffe verwendet:

25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

## Biologische Beispiele

### Beispiel 6

Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95 – 100% relativer Luftfeuchtigkeit und ca. 20° C wurden die infizierten Pflanzen in einem Gewächshaus bei ca. 22° C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz, bezogen auf das Bodenvolumen). Nach 48 Stunden wurden die behandelten Pflanzen mit einer Uredosporensuspension des Pilzes infiziert. Nach einer Inkubation während 48 Stunden bei 95 – 100% relativer Luftfeuchtigkeit und ca. 20° C wurden die infizierten Pflanzen in einem Gewächshaus bei ca. 22° C aufgestellt. Die Beurteilung der Rostpustelnentwicklung erfolgte 12 Tage nach der Infektion.

### Beispiel 7

Wirkung gegen Cercospora arachidicola auf Erdnußpflanzen

Residual-protektive Wirkung

10 – 15 cm hohe Erdnußpflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21° C und hoher Luftfeuchtigkeit inkubiert und anschließend bis zum Auftreten der typischen Blattflecken in einem Gewächshaus aufgestellt. Die Beurteilung der fungiziden Wirkung erfolgte 12 Tage nach der Infektion, basierend auf Anzahl und Größe der auftretenden Flecken.

Beispiel 8

Wirkung gegen Botrytis cinerea auf Puffbohnen

Residual-protektive Wirkung

Circa 10 cm hohe Puffbohnenpflanzen wurden mit einer aus Spritzpulver des Wirkstoffes hergestellten Spritzbrühe (0,02% Aktivsubstanz) besprüht. Nach 24 Stunden wurden die behandelten Pflanzen mit einer Konidiensuspension des Pilzes infiziert. Nach einer Inkubation der infizierten Pflanzen während 2—3 Tagen bei 95—100% relativer Luftfeuchtigkeit und 21°C erfolgte die Beurteilung des Pilzbefalls.

Beispiel 9

Wirkung gegen Piricularia oryzae auf Reispflanzen

a) Residual-protektive Wirkung

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95—100% relativer Luftfeuchtigkeit und 24° C wurde der Pilzbefall beurteilt.

b) Systemische Wirkung

Zu zweiwöchigen Reispflanzen wurde eine aus Spritzpulver des Wirkstoffes hergestellte Spritzbrühe gegossen (0,006% Aktivsubstanz, bezogen auf das Erdvolumen). Darauf wurden die Töpfe mit Wasser so weit gefüllt, daß die untersten Stengelteile der Reispflanzen im Wasser standen. Nach 48 Stunden wurden die behandelten Reispflanzen mit einer Konidiensuspension des Pilzes infiziert. Nach einer Inkubation der infizierten Pflanzen während 5 Tagen bei 95—100% relativer Luftfeuchtigkeit und ca. 24° C wurde der Pilzbefall beurteilt.

Beispiel 10

Wirkung gegen Tilletia caries

Tilletiasporen wurden in einer Spritzbrühe, enthaltend 600 ppm Wirkstoff, während 15 min suspendiert. Das Sporen-Substanz-Gemisch wurde tropfenweise auf die Oberfläche von feingesiebter, angefeuchteter Erde in Petrischalen pipettiert. Die so zubereiteten Erdschalen wurden bei hoher Luftfeuchtigkeit und einer Temperatur von 20°C aufgestellt. Nach ca. 10 Tagen wurde die Sporenkeimung unter der Lupe beurteilt. Die Wirkung der Testsubstanzen wurde auf Grund der Anzahl und der Länge der Keimschläuche ermittelt.

Beispiel 11

Wirkung gegen Plasmopara viticola auf Reben

Residual-protektive Wirkung

Im 4—5-Blattstadium wurden Rebensämlinge mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Nach einer Inkubation während 6 Tagen bei 95—100% relativer Luftfeuchtigkeit und 20° C wurde der Pilzbefall beurteilt.

Beispiel 12

Wirkung gegen Venturia inaequalis auf Apfelbäume

Residual-protektive Wirkung

Apfelsämlinge mit ca. 5 entwickelten Blättern wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24°C aufgestellt. Der Schorfbefall wurde 15 Tage nach der Infektion beurteilt.

Die erfindungsgemäßen Verbindungen zeigten im allgemeinen eine gute fungizide Wirkung in den vorhergehenden Versuchen. Unter anderem erreichten die nachfolgend aufgeführten Verbindungen eine Hemmung des Befalls auf weniger als 20% im Vergleich mit unbehandelten, aber infizierten Kontrollpflanzen.

Bei Puccinia graminis: Nr. 1.2, 1.3, 1.4, 1.5, 2.2, 2.5, 2.9, 2.10, 2.11, 2.12, (i), (iii), (iv).
Bei Cercospora arachidicola: Nr. 2.3, 2.5, 2.6, 2.7, 2.9, 2.10, (i), (iv).
Bei Botrytis cinerea: Nr. 1.3, 1.4, 1.5, 2.5.
Bei Piricularia oryzae: Nr. 1.3, 1.4, 2.1, 2.9, 2.11, 2.14, (iv).
Bei Tilletia caries: Nr. 2.5.
Bei Plasmopara viticola: Nr. 1.4, 2.1.
Bei Venturia inaequalis: Nr. 1.4, 2.1.

Beispiel 13

Wirkung gegen Xanthomonas oryzae auf Reis

a) Residual-protektive Wirkung

Reispflanzen der Sorte »Caloro« oder »S6« wurden nach 3wöchiger Anzucht im Gewächshaus mit der Prüfsubstanz in Form einer Spritzbrühe (0,06% Aktivsubstanz) besprüht. Nach eintägigem Antrocknen dieses Spritzbelages wurden die Pflanzen in einem Klimaraum bei 24°C und 75–85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10tätiger Inkubation in demselben Raum wurden die angeschnittenen Blätter welk, rollten sich ein und wurden nekrotisch. Das Ausmaß dieser Krankheitssymptome diente zur Beurteilung der residualen Wirksamkeit der Prüfsubstanz.

b) Systemische Wirkung

In Blumentöpfen gezogene Reispflanzen der Sorte »Caloro« oder »S6« wurden nach 3wöchiger Anzucht mit einer Suspension der Prüfsubstanz gegossen (0,006% Aktivsubstanz, bezogen auf das Erdvolumen). Drei Tage nach dieser Behandlung wurden die Pflanzen in einem Klimaraum bei 24°C und 75–85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10tätiger Inkubation in demselben Raum wurden die angeschnittenen Blätter der Kontrollpflanzen welk, rollten sich ein und wurden nekrotisch. Das Ausmaß dieser Krankheitssymptome an den Prüfpflanzen diente zur Beurteilung der systemischen Wirksamkeit der Prüfsubstanz.

In den obigen Versuchen 13a und 13b zeigten die Verbindungen der Formeln I und IV eine gute Wirkung. Die behandelten Pflanzen zeigten kaum Welke-Symptome oder Nekrose.

Beispiel 14

Wirkung gegen Xanthomonas vesicatoria auf Paprika

a) Residual-protektive Wirkung

Paprikapflanzen der Sorte »California Wonder« wurden nach 3wöchiger Anzucht im Gewächshaus mit der Prüfsubstanz in Form einer Spritzbrühe (0,06% Aktivsubstanz) besprüht. Nach eintägigem Antrocknen dieses Spritzbelages wurden die Pflanzen in einem Klimaraum bei 26°C und 95–100%

relativer Luftfeuchtigkeit aufgestellt und durch Besprühen der Blattunterseiten mit einer standardisierten Suspension von Xanthomonas vesicatoria infiziert. Nach 6tägiger Inkubation in demselben Raum entstanden auf den Blättern der Kontrollpflanzen runde, anfangs wäßrige, später nekrotische, aufgehellte Flecken. Das Ausmaß dieser Flecken an Prüfpflanzen diente zur Beurteilung der residualen Wirksamkeit der Prüfsubstanz.

b) Systemische Wirkung

In Blumentöpfen gezogene Paprikapflanzen der Sorte »California Wonder« wurden nach 3wöchiger Anzucht mit einer Suspension der Prüfsubstanz gegossen (0,006% Aktivsubstanz, bezogen auf das Erdvolumen). Drei Tage nach dieser Behandlung wurden die Pflanzen in einem Klimaraum bei 26°C und 95–100% relativer Luftfeuchtigkeit aufgestellt und durch Besprühen der Blattunterseiten mit einer standardisierten Suspension von Xanthomonas vesicatoria infiziert. Nach 6tägiger Inkubation in demselben Raum entstanden auf den Blättern der Kontrollpflanzen runde, anfangs wäßrige, später nekrotische, aufgehellte Flecken. Das Ausmaß dieser Flecken diente zur Beurteilung der systemischen Wirksamkeit der Prüfsubstanz.

In den obigen Versuchen 14a und 14b zeigten unter anderem die Verbindungen 1.2, 1.3, (iii), (iv) eine gute Wirkung.

**Patentansprüche**

1. 2,3-Dichlor-7-hydroxy-1H-inden-1-on-Derivate der Formel

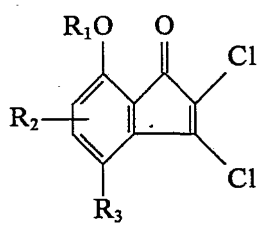

(I)

worin $R_1$ für Wasserstoff oder eine der Gruppen

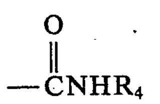 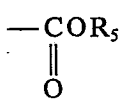 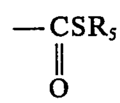 oder 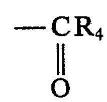

steht und $R_2$ und $R_3$ unabhängig voneinander Wasserstoff, Fluor, Chlor, Brom, $C_1-C_4$-Alkyl, Trifluormethyl oder Nitro, $R_4$ Wasserstoff, unsubstituiertes oder durch Halogen substituiertes $C_1-C_6$-Alkyl, unsubstituiertes oder durch Halogen substituiertes $C_2-C_4$-Alkenyl, unsubstituiertes oder durch $C_1-C_4$-Alkyl, $C_1-C_4$-Alkoxy, Halogen, Trifluormethyl, Cyano oder Nitro substituiertes Phenyl oder aber eine unsubstituierte $C_3-C_6$-Cycloalkylgruppe und $R_5$ $C_1-C_6$-Alkyl, $C_3-C_6$-Cycloalkyl oder $C_2-C_4$-Alkenyl bedeuten.

2. Verbindungen gemäß Anspruch 1, worin in der Formel I $R_2$ für Wasserstoff oder in 6-Stellung Nitro, Fluor, Chlor oder Brom oder in 5-Stellung Fluor, Chlor oder Brom, $R_3$ für Wasserstoff, Fluor, Chlor, Brom oder $C_1-C_4$-Alkyl, $R_4$ für gegebenenfalls halogeniertes $C_1-C_4$-Alkyl oder $C_2-C_3$-Alkyl und $R_5$ für $C_1-C_4$-Alkyl stehen.

3. Verbindungen gemäß Anspruch 1 oder 2, worin in der Formel I $R_1$ für Wasserstoff oder

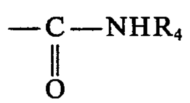

steht.

4. Verbindungen gemäß einem der Ansprüche 1 bis 3, worin in der Formel I $R_2$ für Wasserstoff oder in 6-Stellung Nitro oder Halogen steht.

5. Verbindungen gemäß einem der Ansprüche 1 bis 4, worin in der Formel I $R_4$ Methyl oder Äthyl bedeutet.

6. Eine Verbindung aus der Gruppe

2,3-Dichlor-4-methyl-7-hydroxy-1H-inden-1-on;
2,3-Dichlor-4-fluor-7-hydroxy-1H-inden-1-on;
4-Chlor-2,3-dichlor-7-hydroxy-1H-inden-1-on;
4-Brom-2,3-dichlor-7-hydroxy-1H-inden-1-on;
2,3-Dichlor-4-methyl-7-methylcarbamoyloxy-1H-inden-1-on;
4-Chlor-2,3-dichlor-7-acetoxy-1H-inden-1-on; und
7-Chloracetoxy-2,3-dichlor-4-fluor-1H-inden-1-on.

7. Mikrobizide Mittel, enthaltend als aktive Komponente mindestens eine Verbindung gemäß einem der Ansprüche 1 bis 6, zusammen mit geeigneten Trägermaterialien.

8. Verwendung von Verbindungen gemäß einem der Ansprüche 1 bis 6 zur Bekämpfung von Mikroorganismen.

9. Verfahren zur Bekämpfung von Mikroorganismen, dadurch gekennzeichnet, daß man die zu bekämpfenden Mikroorganismen oder ihren Lebensbereich mit einer Verbindung gemäß einem der Ansprüche 1 bis 6 oder mit einem Mittel gemäß Anspruch 7 behandelt.

10. Verwendung gemäß Anspruch 8 oder Verfahren gemäß Anspruch 9, wobei die zu bekämpfenden Mikroorganismen phytopathogene Pilze sind.

11. Verwendung gemäß Anspruch 8 oder Verfahren gemäß Anspruch 9, wobei die zu bekämpfenden Mikroorganismen phytopathogene Bakterien sind.

12. Verfahren zur Herstellung von Verbindungen gemäß Anspruch 1, dadurch gekennzeichnet, daß man

a) eine Verbindung der Formel II

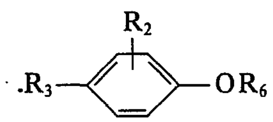

(II)

worin $R_2$ und $R_3$ die für Formel I gegebene Bedeutung haben und $R_6$ Wasserstoff oder Methyl darstellt, mit Trichloracrylsäurechlorid der Formel

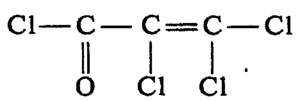

(III)

in Gegenwart einer Lewis-Säure umsetzt und

b) die so erhaltene Verbindung der Formel IV

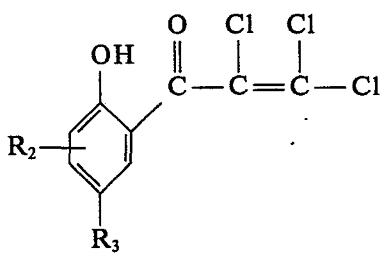

(IV)

ebenfalls in Gegenwart einer Lewis-Säure zu einer Verbindung der Formel V

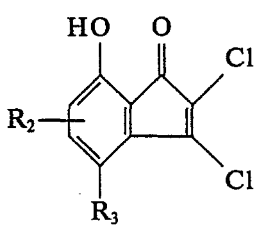

(V)

umsetzt, die einer Verbindung der Formel I entspricht, worin $R_1$ Wasserstoff bedeutet und

c) zur Herstellung von Verbindungen der Formel I, worin $R_1$ eine andere Bedeutung als Wasserstoff hat, diese Verbindung der Formel V mit einem, der Bedeutung von $R_1$ entsprechenden Säurehalogenid, Isocyanat, Halogenameisensäureester oder Halogenthioameisensäureester umsetzt.

13. Verfahren gemäß Anspruch 12, wobei $R_6$ in der Formel II Methyl bedeutet.

14. Verbindungen der Formel IV

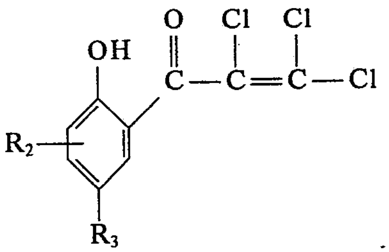

(IV)

worin $R_2$ und $R_3$ die im Anspruch 1 gegebenen Bedeutungen haben.

15. Mikrobizide Mittel, enthaltend als aktive Komponente mindestens eine Verbindung gemäß Anspruch 14.

16. Verwendung von Verbindungen gemäß Anspruch 14 zur Bekämpfung von Mikroorganismen.

## Claims

1. A 2,3-dichloro-7-hydroxy-1H-inden-1-one derivative of the formula

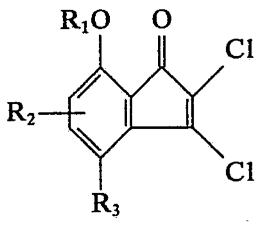

(I)

wherein

$R_1$ is hydrogen or one of the groups

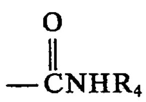 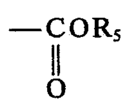 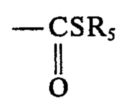 or 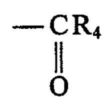

$R_2$ and $R_3$ independently of one another are each hydrogen fluorine, chlorine, bromine, $C_1-C_4$-alkyl, trifluoromethyl or nitro,

$R_4$ is hydrogen, $C_1-C_6$-alkyl or $C_2-C_4$-alkenyl, each of which is unsubstituted or substituted by halogen, or $R_4$ is phenyl which is unsubstituted or is substituted by $C_1-C_4$-alkyl, $C_1-C_4$-alkoxy, halogen, trifluoromethyl, cyano or nitro, or it is a $C_3-C_6$-cycloalkyl group, and

$R_5$ is $C_1-C_6$-alkyl, $C_3-C_6$-cycloalkyl or $C_2-C_4$-alkenyl.

2. A compound according to Claim 1 wherein in formula I $R_2$ is hydrogen, or in the 6-position it is nitro, fluorine, chlorine or bromine, or in the 5-position fluorine, chlorine or bromine; $R_3$ is hydrogen, fluorine, chlorine, bromine or $C_1-C_4$-alkyl; $R_4$ is $C_1-C_4$-alkyl or $C_2-C_3$-alkyl, each of which is unsubstituted or substituted by halogen; and $R_5$ is $C_1-C_4$-alkyl.

3. A compound according to Claim 1 or 2, wherein in the formula I $R_1$ is hydrogen or

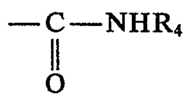

4. A compound according to any one of Claims 1 to 3, wherein in the formula I $R_2$ is hydrogen, or in the 6-position it is nitro or halogen.

5. A compound according to any one of Claims 1 to 4, wherein in the formula I $R_4$ is methyl or ethyl.

6. A compound from the group

2,3-dichloro-4-methyl-7-hydroxy-1H-inden-1-one;
2,3-dichloro-4-fluoro-7-hydroxy-1H-inden-1-one;

4-chloro-2,3-dichloro-7-hydroxy-1H-inden-1-one;
4-bromo-2,3-dichloro-7-hydroxy-1H-inden-1-one;
2,3-dichloro-4-methyl-7-methylcarbamoyloxy-1H-inden-1-one;
4-chloro-2,3-dichloro-7-acetoxy-1H-inden-1-one; and
7-chloroacetoxy-2,3-dichloro-4-fluoro-1H-inden-1-one.

7. A microbicidal composition containing as active ingredient at least one compound according to any one of Claims 1 to 6 inclusive, together with suitable carriers.

8. Use of a compound according to any one of Claims 1 to 6 inclusive for combating microorganisms.

9. A process for combating microorganisms, which process comprises treating the microorganisms to be combated, or the habitat thereof, with a compound according to any one of Claim 1 to 6 inclusive, or with a composition according to Claim 7.

10. Use according to Claim 8 or process according to Claim 9, whereby the microorganisms to be combated are phytopathogenic fungi.

11. Use according to Claim 8 or process according to Claim 9, whereby the microorganisms to be combated are phytopathogenic bacteria.

12. A process for producing a compound according to Claim 1, which process comprises reacting

a) a compound of the formula II

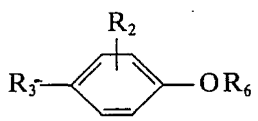

(II)

wherein $R_2$ and $R_3$ have the meanings given under the formula I, and $R_6$ is hydrogen or methyl, with trichloroacrylic acid chloride of the formula

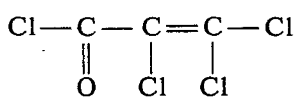

(III)

in the presence of a Lewis acid; and

b) reacting the resulting compound of the formula IV

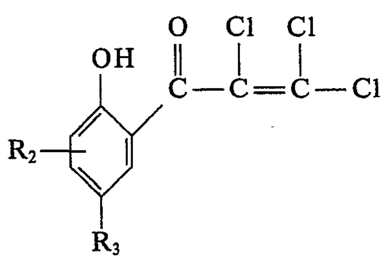

(IV)

likewise in the presence of a Lewis acid, to give a compound of the formula V

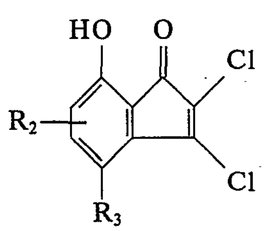

(V)

which corresponds to a compound of the formula I wherein $R_1$ is hydrogen; and

c) in order to produce a compound of the formula I wherein $R_1$ has a meaning other than hydrogen, reacting the compound of the formula V with an acid halide, isocyanate, haloformic acid ester or halothioformic acid ester, corresponding in each case to the meaning of $R_1$.

13. A process according to Claim 12, wherein $R_6$ in the formula II is methyl.

14. A compound of the formula IV

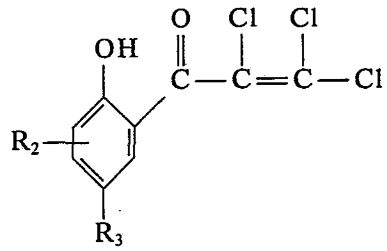

(IV)

wherein $R_2$ and $R_3$ have the meanings given in Claim 1.

15. A microbicidal composition containing as active ingredient at least one compound according to Claim 14.

16. Use of a compound according to Claim 14 for combating microorganisms.

**Revendications**

1. Dérivés de la 2,3-dichloro-7-hydroxy-1H-indène-1-one de formule:

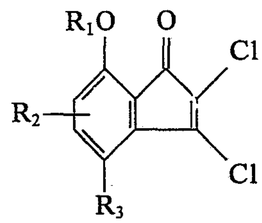

(I)

dans laquelle

$R_1$ représente l'hydrogène ou l'un des groupes

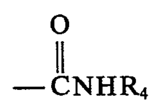 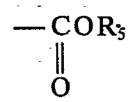 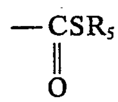 ou 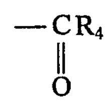

$R_2$ et $R_3$ représentent, indépendamment l'un de l'autre, l'hydrogène, le fluor, le chlore, le brome, un groupe alkyle en $C_1-C_4$, trifluorométhyle ou nitro,

$R_4$ représente l'hydrogène, un groupe alkyle en $C_1-C_6$ éventuellement halogéné ou alcényle en $C_2-C_4$ éventuellement halogéné, un groupe phényle éventuellement substitué par des groupes alkyles en $C_1-C_4$, alcoxy en $C_1-C_4$, des halogènes, des groupes trifluorométhyle, cyano ou nitro ou encore un groupe cycloalkyle en $C_3-C_6$ et

$R_5$ représente un groupe alkyle en $C_1-C_6$, cycloalkyle en $C_3-C_6$ ou alcényle en $C_2-C_4$.

2. Composés selon la revendication 1, pour lesquels, dans la formule (I)

$R_2$ représente l'hydrogène ou, en position 6, un groupe nitro, le fluor, le chlore ou le brome, ou, en position 5, le fluor, le chlore ou le brome,

$R_3$ représente l'hydrogène, le fluor, le chlore ou le brome ou un groupe alkyle en $C_1-C_4$,

$R_4$ représente un groupe alkyle en $C_1-C_4$ éventuellement halogéné ou un groupe alkyle en $C_2-C_3$ et

$R_5$ représente un groupe alkyle en $C_1-C_4$.

3. Composés selon la revendication 1 ou 2, pour lesquels, dans la formule (I), $R_1$ représente l'hydrogène ou le groupe

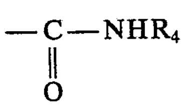

4. Composés selon l'une des revendications 1 à 3, pour lesquels, dans la formule (I), $R_2$ représente l'hydrogène ou, en position 6, un groupe nitro ou un halogène.

5. Composés selon l'une des revendications 1 à 4, pour lesquels, dans la formule (I), $R_4$ représente un groupe méthyle ou éthyle.

6. Un composé du groupe:

2,3-dichloro-4-méthyl-7-hydroxy-1H-indène-1-one
2,3-dichloro-4-fluoro-7-hydroxy-1H-indène-1-one
4-chloro-2,3-dichloro-7-hydroxy-1H-indène-1-one
4-bromo-2,3-dichloro-7-hydroxy-1H-indène-1-one
2,3-dichloro-4-méthyl-7-méthylcarbamoyloxy-1H-indène-1-one
4-chloro-2,3-dichloro-7-acétoxy-1H-indène-1-one et
7-chloracétoxy-2,3-dichloro-4-fluoro-1H-indène-1-one.

7. Produit microbicide contenant en tant que composant actif au moins un composé selon l'une des revendications 1 à 6, avec des véhicules appropriés.

8. Utilisation des composés selon l'une des revendications 1 à 6 dans la lutte contre les microorganismes.

9. Procédé pour combattre les microorganismes, caractérisé en ce que l'on traite les microorganismes à combattre ou leur espace vital par un composé selon l'une des revendications 1 à 6 ou par un produit selon la revendication 7.

10. Utilisation selon la revendication 8 ou procédé selon la revendication 9, dans lesquels les microorganismes à combattre sont des mycètes phytopathogènes.

11. Utilisation selon la revendication 8 ou procédé selon la revendication 9, dans lesquels les microorganismes à combattre sont des bactéries phytopathogènes.

12. Procédé de préparation des composés selon la revendication 1, caractérisé en ce que:

a) on fait réagir un composé de formule (II):

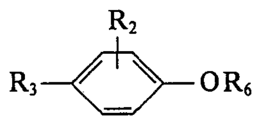

(II)

dans laquelle
$R_2$ et $R_3$ ont les significations indiquées en référence à la formule (I) et
$R_6$ représente l'hydrogène ou le groupe méthyle, avec le chlorure de l'acide trichloracrylique de formule:

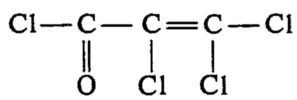

(III)

en présence d'un acide de Lewis et

b) on convertit le composé ainsi obtenu répondant à la formule (IV):

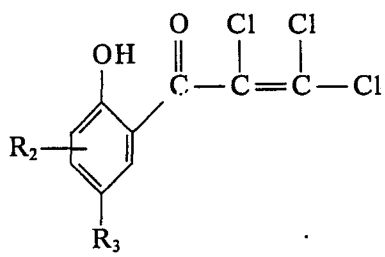

(IV)

également en présence d'un acide de Lewis, en un composé de formule (V):

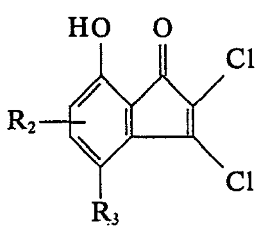

(V)

qui correspond à un composé de formule (I) dans laquelle $R_1$ représente l'hydrogène, et

c) pour préparer les composés de formule (I) dans laquelle $R_1$ a une signification autre que l'hydrogène, on fait réagir ce composé de formule (V) avec un halogénure d'acide, isocyanate, ester halogénoformique ou ester halogénothioformique correspondant à la signification de $R_1$.

13. Procédé selon la revendication 12, dans lequel $R_6$ de la formule (II) représente un groupe méthyle.

14. Composés de formule (IV):

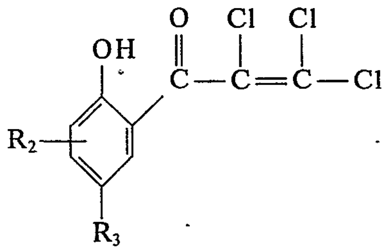

(IV)

dans laquelle $R_2$ et $R_3$ ont les significations indiquées dans la revendication 1.

15. Produit microbicide contenant, en tant que composant actif, au moins un composé selon la revendication 14.

16. Utilisation des composés selon la revendication 14 dans la lutte contre les microorganismes.